# EUROPÄISCHE PATENTSCHRIFT

(11) **EP 2 468 178 B1**
(45) Veröffentlichungstag und Bekanntmachung des Hinweises auf die Patenterteilung: **14.12.2016**
(21) Anmeldenummer: 12001041.8
(22) Anmeldetag: 02.06.2004
(51) Int. Cl.: A61B 5/00, A61B 5/15, G01N 33/487

(54) **Integriertes Testelement zur einmaligen Aufnahme und Analyse einer zu untersuchenden Probe**
Integrated test element for one-off holding and analysis of a sample to be tested
Elément de test intégré destiné au prélèvement en une fois et à l'analyse d'un échantillon à analyser

(30) Priorität: 06.06.2003 DE 10325699
(43) Veröffentlichungstag der Anmeldung: 27.06.2012
(62) Teilanmeldung aus: 04739508.2
(73) Patentinhaber: Roche Diabetes Care GmbH, 68305 Mannheim (DE); F.Hoffmann-La Roche AG, 4070 Basel (CH)
(72) Erfinder: Hoenes, Joachim, 64673 Zwingenberg (DE); Miltner, Karl, 67227 Frankenthal (DE)
(74) Vertreter: Silber, Anton

(56) Entgegenhaltungen:
- WO-A-99/07277
- WO-A-02/100274
- WO-A1-01/66010
- US-A- 4 622 974
- US-A- 5 636 640
- US-A- 6 032 059

## Beschreibung

Die Erfindung bezieht sich auf das Gebiet von integrierten Testelemente, die zur Aufnahme einer Probe, vorzugsweise Blut, über ein Stechinstrument verfügen, mit dem zunächst eine Wunde in einer Hautöffnung erzeugt werden kann. Das austretende Blut wird anschließend von dem Testelement aufgenommen, wobei es mit einem im Testelement enthaltenem Reagenz in Kontakt kommt und eine optisch detektierbare Veränderung in einem Testfeld bewirkt. Die Veränderung im Testfeld wird mittels einer Analyseeinheit detektiert, so dass ein im Blut enthaltener Analyt bestimmt werden kann.

Auf medizinischem Gebiet kommt als Probe vor allem Blut in Betracht. Nachfolgend wird ohne Beschränkung der Allgemeinheit beispielhaft auf die Blutanalyse Bezug genommen. Ein besonders wichtiges Anwendungsgebiet bei der Blutanalyse ist die Kontrolle des Blutzuckerspiegels von Diabetikern, die insbesondere im Analysebereich der Blutzuckerselbstkontrolle ("home monitoring") häufig eingesetzt wird.

In diesem Anwendungsgebiet werden im großem Umfang photometrische, trägergebundene Tests gebraucht. Derartige Testelemente werden häufig als Einmalartikel verwendet, die üblicherweise ein Reagenzsystem enthalten, das irreversibel mit einem Analyten einer Probe reagiert und eine charakteristische, optisch messbare Veränderung des Testelementes bewirkt.

Herkömmliche Testelemente, die bei photometrischen Tests verwendet werden, haben meist die Form der im Stand der Technik bekannten Teststreifen, auf denen ein Testfeld aufgebracht ist. Das Testfeld besteht aus einem Reagenzsystem, das unterschiedliche Funktionen erfüllen kann. Die Probe wird auf die Oberseite des Testfelds aufgegeben. Nach Ablauf einer erforderlichen Reaktionszeit werden zur Analyse der Probe charakteristische Farbänderungen mit Hilfe einer Analyseeinheit reflektionsphotometrisch vermessen. Das Auswertegerät, das zur Auswertung eines Analyseergebnisses vorgesehen ist, ist in der Regel für einen ganz bestimmten Typ von Testelementen eines bestimmten Herstellers geeignet. Die Testelemente und das Auswertegerät bilden somit wechselseitig aufeinander abgestimmte Bestandteile und werden üblicherweise insgesamt als Analysesystem bezeichnet. Derartige Analysesysteme werden beispielsweise in dem US-Patent 5,281,395 und 5,424,035 beschrieben.

Zur Entnahme einer Blutprobe, insbesondere im Home-Monitoring-Bereich werden zusätzlich zu den Analysesystemen Stechhilfen gehandelt, mit deren Hilfe der Patient eine Hautöffnung erzeugen kann. Das durch die Hautöffnung austretende Blut kann auf ein Testelement aufgegeben werden. Bei dem beschriebenen System ist der Benutzer auf ein komplexes Handling angewiesen, bei dem er zuerst eine Wunde erzeugen muss, um über ein hinreichendes Probenvolumen zur Blutanalyse verfügen zu können. Der Ort des Blutaustrittes wird anschließend mit dem Testfeld eines Testelementes in Kontakt gebracht, so dass hinreichend Probe in das Testfeld eindringen kann. Nachfolgend muss das Testfeld mit der Probe relativ zur Analyseeinheit positioniert werden, um eine Analyse des Testfeldes zu ermöglichen.

Um den beschriebenen komplexen Ablauf für den Benutzer zu vereinfachen, werden im Stand der Technik so genannte integrierte Systeme angeboten, die mehrere Arbeitsschritte zusammenfassen und somit das Handling zur Probenanalyse auf einige wenige Handhabungsschritte reduzieren.

In dem Dokument WO 97/42888 wird ein Blutentnahmesystem beschrieben, mit dem zum einen eine Wunde in einem Körperteil erzeugt werden kann, so dass Blut zur Analyse aus der Körperöffnung hervortritt. Zum anderen ist das Blutentnahmesystem gleichzeitig so beschaffen, dass eine Kanüle im System, die nahe der Lanzette und somit nahe dem Einstichort angeordnet ist, zur Blutaufnahme geeignet ist. Die Probe kann somit nach dem Stechvorgang, bevorzugt durch Kapillareffekte, in die Kanüle aufgesaugt werden und anschließend auf ein hierfür vorgesehenes Testelement mit einem Reagenzsystem abgegeben werden. Die hierbei verwendeten Testelemente sind wie im Stand der Technik hinlänglich bekannt ausgestaltet und werden entsprechend angewendet. Obwohl dem Benutzer bei dem beschriebenen System die Probenaufgabe auf das Testelement erleichtert wird, sind jedoch separate Bedienungsschritte durch den Benutzer erforderlich, die das Blut aus der Kanüle heraus auf ein Testfeld übertragen.

Das Dokument US 4,360,016 offenbart ein analoges Blutentnahmesystem, bei dem der Stechvorgang und die Probeentnahme ebenfalls in einem System integriert sind. Der beschriebene Stand der Technik erweist sich jedoch neben den aufgeführten Handhabungsschritten weiterhin als nachteilig, da eine erhöhte Probenmenge im System benötigt wird. Hierbei muss auch nach der Aufnahme und Abgabe des Blutes durch eine Kanüle ein hinreichendes Probenvolumen zur Aufgabe auf das Testfeld zur Verfügung stehen.

In dem Patent US 4,627,445 wird ein Analysesystem beschrieben, bei dem die Funktionen Stechen, Probeaufnahme, Probenaufgabe auf das Testfeld und Analyse in einem System integriert sind. Hierfür wird zunächst mittels einer Lanzette eine Hautöffnung erzeugt, aus der das Blut austreten kann. Aufgrund eines im System vorherrschenden Unterdrucks wird das Blut von der Blutaustrittsstelle über einen seitlich angeordneten Kanal in das Analysesystem aufgesogen und zu einem Testfeld hingeleitet. Das Testfeld ist dabei so im Analysesystem angeordnet, dass eine Messeinheit, die direkt oberhalb des Testfeldes positioniert ist, das Testfeld auswerten kann.

In dem Patent US 6,032,059 wird ein Analysesystem beschrieben, bei dem ein Testfeld am Ende eines lichtleitenden Elements in einer Lanzette angeordnet ist.

Nachteil des dargestellten Standes der Technik ist der sehr komplexe Aufbau des Analysesystems, der unter anderem einen Unterdruck zur Probenaufnahme benötigt. Des weiteren muss auch hier hinreichend Probenvolumen aus der Körperöffnung austreten, damit eine vollständige Bedeckung des Testfeldes nach der Überführung der Probe in den Transportkanal hin zum Testfeld gegeben ist.

Prinzipiell muss, um eine hinreichende Probenmenge für derartige Systeme - trotz gegebener Totvolumina in den Transportkanälen - erzielen zu können, die Einstichtiefe der Lanzette entsprechend tief und somit die Wunde hinreichend groß gewählt werden. Mit einer Vergrößerung der Einstichtiefe resultiert jedoch ein erhöhtes Schmerzempfinden, was insbesondere bei Patienten, die sich mehrfach am Tag Blut entnehmen müssen, vermieden werden sollte.

Aufgabe der Erfindung ist es, die beschriebenen Nachteile des Standes der Technik zu vermeiden. Das erfindungsgemäße Blutentnahmesystem soll hierbei ein komfortables Handling für den Benutzer ermöglichen, ohne dass hierdurch bedingt eine vergrößerte Menge Probenvolumen erforderlich ist.

Die Erfindung wird durch die unabhängigen Ansprüche beschrieben. Bevorzugte Ausführungsformen der Erfindung ergeben sich gemäß der abhängigen Ansprüche.

Die Erfindung wird charakterisiert durch ein System, das zur Aufnahme und Analyse einer zu untersuchenden Probe geeignet ist. Das System beinhaltet ein Testfeld mit einem Reagenz, das bei Kontakt mit dem in der Probe enthaltenen Analyten reagiert und eine optisch detektierbare Veränderung im Testfeld bewirkt. Das System beinhaltet weiterhin eine Lanzette mit einer Lanzettenspitze. Erfindungsgemäß weist das System mindestens ein lichtleitendes Element auf. Das lichtleitende Element ist mit einem ersten distalem Ende im Bereich des Testfeldes angeordnet, wobei in ein zweites proximales Ende des lichtleitenden Elementes Licht eingekoppelt werden kann, so dass Licht von dem zweiten Ende zu dem Testfeld hingeleitet und von dem oder einem weiterem lichtleitendem Element wieder vom Testfeld weggeleitet werden kann. Hierbei sind die Lanzette, das Testfeld und das mindestens eine lichtleitende Element so zueinander angeordnet, dass die Lanzettenspitze während eines Stechvorgangs über das distale Ende des Lichtleiters sowie über das Testfeld hinausragt. Zur Aufnahme der Probe wird das Testfeld mit der Probe in Kontakt gebracht, wobei das distale Ende des lichtleitenden Elementes im wesentlichen direkt an dem Probenaufnahmeort positioniert ist.

Durch das erfindungsgemäße System ist es möglich, die Probe am Probenaufnahmeort des Systems direkt durch das Testfeld aufzunehmen, ohne dass hierzu zusätzliche Transportkanäle benötigt werden. Der Probeaufnahmeort des Systems wird folglich durch das Testfeld realisiert, so dass kein Transport der Probe zu einem Testfeld im System erfolgen muss. Die Position des Testfeldes und somit des Probeaufnahmeortes relativ zu einer Analyseeinheit in einem Analysesystem kann dabei beliebig gewählt werden und sollte an einem für den Benutzer leicht zugängliche Ort vorgesehen sein. Die optische Kontaktierung zwischen einer Analyseeinheit zur Vermessung des Testfeldes und dem Testfeld wird dabei erfindungsgemäß über mindestens ein lichtleitendes Element realisiert, so dass eine relative Positionierung von Testfeld und Analyseeinheit entsprechend flexibel ausgestaltet werden kann. Auf diese Weise kann zum einen das zur Analyse benötigte Probenvolumen reduziert werden, da keine Totvolumina durch Transportkanäle bedingt werden, zum anderen kann der Aufbau eines Analysesystems, das zur Verwendung des erfindungsgemäßen Systems geeignet ist, entsprechend komfortabel den Bedürfnissen des Benutzers angepasst werden.

Durch die Integration eines lichtleitenden Elementes in das erfindungsgemäße System kann das Testfeld, sofern das lichtleitende Element mit einer Analyseeinheit optisch kontaktiert ist, direkt vermessen werden, ohne dass eine Positionierung des Testfeldes nach der Probenaufnahme relativ zu einer Optik in einem Analysegerät durch den Benutzer erfolgen muss. Eine Auswertung des Testfeldes mittels eines hierzu geeigneten Analysegerätes ist somit auch außerhalb des Analysegerätes möglich, so dass ein sog. "outside dosing" für eine integrierte photometrische Analyse ermöglich wird. Hierbei befindet sich in der Regel ein erster Teilabschnitt des lichtleitenden Systems innerhalb des Gerätes, während ein zweiter Teilabschnitt, das distale Ende des Lichtleiters, aus dem Gerät herausragt und dadurch für den Benutzer leicht zugänglich ist. Verschmutzungen des Gerätes bei der Blutaufgabe auf das am distalen Ende positionierte Testfeld, können somit vermieden werden. Darüber hinaus kann der Aufbau eines Analysesystems stark vereinfacht werden, da an die Positionierung der Optik im Analysesystem keine besonderen Anforderungen gestellt werden. Das System kann, wie es im Stand der Technik im wesentlichen bekannt ist, an das Analysegerät in herkömmlicher Weise angekoppelt werden, ohne dass komplexe oder ungewohnte Handhabungsschritte durch den Benutzer erforderlich sind. Erfindungsgemäß findet hierbei eine optische Kontaktierung zwischen System und Analysegerät statt, so dass eine Positionierung des Testfeldes relativ zur Optik automatisch gewährleistet ist.

Im Rahmen der Erfindung ist der Bereich des Testfeldes, in dem das distale Ende des lichtleitenden Elementes positioniert ist, dadurch gekennzeichnet, dass das Testfeld und das distale Ende so zueinander angeordnet ist, dass eine optische Kontaktierung zwischen Testfeld und lichtleitendem Element möglich ist. Generell ist das System auf keine spezielle Ausführungsform eines Testfeldes beschränkt, wobei erfindungsgemäß die Anordnung von Testfeld und distalem Ende eine Vermessung des Testfeldes durch Licht erlaubt, welches über das lichtleitende Element zum Testfeld transportiert bzw. wieder weggeleitet wird. Dabei kann das Testfeld beispielsweise direkt auf dem lichtleitendem Element aufgebracht sein oder nur an diesem angeordnet werden.

Erfindungsgemäss ist das Testfeld fest mit dem Lichtleiter verbunden und es kann hierfür z. B. eine Reagenzschicht auf das distale Ende des Lichtleiters aufgeklebt, gesprüht oder durch Photopolymerisation aufgebracht werden. Das Vorgehen der Photopolymerisation wird beispielhaft in dem Dokument DE 102 21840.4 beschrieben.

Da an den Aufbau des Testfeldes keine besonderen Anforderungen gestellt werden, können z. B. ein- oder mehrschichtige, aus dem Stand der Technik hinreichend bekannte Testfelder verwendet werden. Erfindungsgemäß erfolgt dabei nach Aufgabe einer Probe auf ein Testfeld eine optisch detektierbare Veränderung des selben. Beispielsweise können auch Testfelder verwendet werden, wie sie aus Teststreifen im Stand der Technik mit Probenvorbereitungsfunktionen bekannt sind. Ein derartiges Testfeld ist mehrschichtig aufgebaut und kann insbesondere dazu dienen, die gleichmäßige Benetzung des Testfeldes mit der Probenflüssigkeit zu fördern. Aufgrund der mehrschichtigen Struktur können z. B. rote Blutkörperchen aus Vollblut in einer oberen Schicht abgetrennt werden, so dass ausschließlich Plasma in einem unteren Bereich des Testfeldes gelangt, in dem eine Reaktion mit einem Reagenz stattfindet. Die Auswertung des Messsignals, d. h. der gemessenen Intensität des vom Testfeld weggeleiteten Lichts und die Ermittlung der gewünschten Analyseresultate, beispielsweise der Glucosekonzentration in der Probe, erfolgt mittels der Mess- und Auswerteelektronik prinzipiell in der gleichen Weise, wie bei üblichen Analysesystemen und muss deshalb an dieser Stelle nicht näher erläutert werden.

Wird die im Testfeld induzierte, optisch detektierbare Veränderung mittels des lichtleitenden Elementes erfasst und von einer Analyseeinheit detektiert und ausgewertet, kann dieses durch eine photometrische Auswertung beispielsweise der vom Testfeld reflektierter Strahlung realisiert werden. Es ist jedoch auch denkbar, dass das lichtleitende Element und das Testfeld so zueinander angeordnet sind, dass eine Messung der optisch detektierbaren Veränderung im Testfeld in Transmission erfolgt. Messanordnung und Testfeld müssen dann entsprechend für eine Transmissionsmessung beschaffen sein. In einer bevorzugten Ausführungsform wird eine Auswertung durch Fluoreszenzmessungen realisiert. Hierbei kann vorteilhafterweise ein einziger Lichtleiter für die Einstrahlung sowie für die Emission benutzt werden. Durch einen geeigneten Filter vor einem Detektor einer Analyseeinheit wird dann die Anregungsstrahlung im wesentlichen soweit eliminiert, dass es zu keinerlei

Überlappungen von Einstrahlung und Emission kommt, so dass eine hohe Messgenauigkeit erzielt wird.

Das lichtleitende Element des Systems besteht Vorteilhafterweise aus einem Material, das im Wellenlängenbereich des eingestrahlten Lichts, das zur Analyse der Probe am zweiten Ende des Lichtleiters eingekoppelt wird, möglichst transparent ist, so dass im wesentlichen keine optische Absorption erfolgt. Vorzugsweise ist der Brechungsindex des Materials größer als der Brechungsindex der Umgebung, so dass innerhalb des lichtleitenden Elementes eine Totalreflektion stattfindet. Prinzipiell ist es auch denkbar, dass das Licht innerhalb des lichtleitenden Elementes aufgrund einer metallischen Reflektion einer das Lichtleiterelement begrenzenden Grenzflächen basiert. Nähere Informationen über lichtleitende Elemente, deren Lichttransporte auf Totalreflektion basieren, können der einschlägigen Literatur entnommen werden. Beispielsweise wird die Verwendung von Lichtleitern in einem Teststreifen in dem Dokument WO 01/48461 beschrieben.

In einer bevorzugten Ausführungsform, wird Licht, im nachfolgenden auch als Primärlicht bezeichnet, unter Bedingung der Totalreflektion zum ersten Ende des lichtleitenden Elementes geleitet, an dem eine Auskopplung des Lichtes in das Testfeld erfolgt. Hierbei wird das Primärlicht beispielsweise direkt auf das Testfeld geleitet oder wird von dem lichtleitenden Element zunächst in das Testfeld umgelenkt. Eine Änderung der Lichtausbreitungsrichtung kann z. B. durch eine spiegelnde Fläche erzielt werden, die z. B. unter einem Winkel von ca. 45° geneigt ist. Beispielsweise kann eine derartig spiegelnde Eigenschaft durch eine metallisch glänzende Beschichtung oder eine polierte Fläche etc. erreicht werden. Prinzipiell können verschiedene Mittel eingesetzt werden, um die gewünschte Auskopplung des Lichtes aus dem lichtleitenden Element in das Testfeld zu bewirken. Insbesondere kann der Brechungsindex der Umgebung und der des lichtleitenden Elementes im Auskopplungsbereich so gestaltet sein, dass im wesentlichen keine Totalreflektion mehr stattfindet. Die Auskopplung kann darüber hinaus dadurch gefördert werden, dass die Oberfläche des lichtleitenden Elementes in dem Auskopplungsbereich aufgeraut ist. Des weiteren kann die Auskopplung des Lichtes durch eine geeignete Lichtführung innerhalb der Lichtleiterschicht bewirkt werden, durch die erreicht wird, dass zumindest große Teile des Primärlichtes in den Auskopplungsbereich unter einem Winkel auf die im Testfeld zugewandten Grenzfläche auftreffen, der größer als der Grenzwinkel der Totalreflektion ist. Beispielsweise wird hierfür das distale Ende des lichtleitenden Elementes so abgeschrägt, dass das Primärlicht in das Testfeld gespiegelt wird.

Das aus dem Testfeld diffus reflektierte, emittierte oder transmittierte Sekundärlicht wird anschließend in das lichtleitende Element eingekoppelt und bevorzugt unter Totalreflektion vom Testfeld wieder weggeleitet. Prinzipiell ist hierbei das lichtleitende Element zumindest abschnittsweise derartig ausgebildet, dass die Lichtausbreitungsrichtung des eingestrahlten Primärlichtes in Richtung auf das Testfeld hin geleitet wird und/oder die Lichtausbreitungsrichtung des von dem Testfeld diffus reflektierten, emittierten oder transmittierten Sekundärlichtes in die zum Detektor führende Richtung des lichtleitenden Elementes gelenkt wird.

Grundsätzlich besteht die Möglichkeit, dass das Primärlicht und das Sekundärlicht in einem lichtleitendem Element transportiert werden. Es hat sich jedoch insbesondere bei Messungen in Reflektion als vorteilhaft erwiesen, mindestens zwei lichtleitende Elemente in das System zu integrieren, so dass Primär- und Sekundärlicht getrennt voneinander transportiert werden können. Werden zwei lichtleitende Elemente verwendet, können Primärlichtleiter und Sekundärlichtleiter optisch weitestgehend getrennt verwirklicht werden. Im Hinblick auf eine optimale Messgenauigkeit erweist sich eine möglichst vollständige optische Trennung von Primärlichtleiter und Sekundärlichtleiter sogar als wichtige Voraussetzung. Zu diesem Zweck kann z. B. eine Lichtsperre vorgesehen werden, die z. B. in Form einer Sperrschicht, deren Brechungsindex kleiner als der Brechungsindex des lichtleitenden Elementes ist, realisiert ist. Des weiteren kann eine optische Trennung durch eine aus metallisch reflektiertem Material ausgebildeten Sperrschicht verwirklicht werden.

Um eine gezielte Einkopplung des Sekundärlichtes in ein zweites lichtleitendes Element zu fördern, ist der Sekundärlichtleiter in einer bevorzugten Ausführungsform abschnittsweise derartig abgeschrägt ist, dass das Sekundärlicht durch eine spiegelnde Fläche in den Sekundärlichtleiter gespiegelt wird. Der Neigungswinkel der spiegelnden Fläche ist ebenfalls vorzugsweise im Winkelbereich von ca. 45°.

Das vom System weggeleitete Sekundärlicht wird von einem Detektor in einem Analysegerät erfasst, so dass eine Bestimmung eines in der Probe enthaltenen Analyten auf Basis des Signals erfolgen kann. Um das Sekundärlicht aus dem lichtleitenden Element in Richtung des Detektors auszukoppeln wird beispielsweise am hinteren Ende des lichtleitenden Elementes wiederum eine schräg verlaufende, spiegelnde Fläche vorgesehen. Statt der spiegelnden Flächen können prinzipiell auch andere Mittel verwendet werden, um die gewünschte Änderung der Lichtausbreitungsrichtung zu bewirken. Insbesondere kann dies durch Brechungsindex-Variation des lichtleitenden Elementes bewirkt werden. Solche Brechungsindex-Variationen können beispielsweise durch Bestrahlung mit UV-Laserlicht erzeugt werden.

Vorteilhafterweise ist das vom Detektor erfasste Licht weitgehend frei von störenden Primärlichtanteilen, so dass ein gutes Signal-Rauschverhältnis erreicht wird.

Erfolgt die Messung in Reflektion, haben Experimente gezeigt, dass im Vergleich zu konventionellen Messungen, in denen keine lichtleitenden Elemente verwendet werden, ein erhöhter Anteil der diffusen Reflektion vom Testfeld des detektierten Sekundärlichtes als Signal erfasst werden kann, so dass eine erhöhte Messgenauigkeit des Verfahrens resultiert. Um die Messgenauigkeit weiterhin zu optimieren, kann das Testfeld darüber hinaus optisch stark streuende Bestandteilen enthalten.

Das System ermöglicht somit neben einer erheblichen Vereinfachung der Handhabungsschritte für den Benutzer sowie ein einfachen Systemaufbau eine erhöhte Messgenauigkeit.

Das erfindungsgemäße System ist als Einmalartikel in einer Stechhilfe zu verwenden, wobei das im Testfeld enthaltende Reagenz mit einem Analyten der Probe im wesentlichen irreversibel reagiert. Die als Reagenz verwendete Chemie ist im Stand der Technik hinreichend bekannt, so dass an dieser Stelle keine nähere Erörterung stattfindet. Beispielsweise wird im Dokument EP 0 821 234 eine Chemie beschrieben, die mit einem Analyten einer Probe irreversibel reagiert. In einer bevorzugten Ausführungsform enthält das Testfeld eine Substanz, die als Reaktion auf den Analyten ihre Fluoreszenz ändern. Derartige Reagenzsysteme werden im Stand der Technik z. B. in Dokument DE 102 21 845.5 beschrieben, auf das an dieser Stelle inhaltlich Bezug genommen wird.

Durch die Verwendung des Systems als Einmalartikel sowie der Möglichkeit des so genannten "outside dosing" können Kontaminationen einer Probe durch vorangegangene Analysen einfach vermieden werden.

Das erfindungsgemäße System wird in einer Stechhilfe verwendet, die eine Antriebseinheit für die Lanzette des Systems aufweist, so dass die Lanzette an die Antriebseinheit angekoppelt und in Einstichrichtung bewegt werden kann.

In einer bevorzugten Ausführungsform ist in der Stechhilfe weiterhin eine Analyseeinheit integriert, die beim Einlegen des Systems in die Stechhilfe mit dem Lichtleiter des Systems optisch kontaktiert wird. Die optische Kontaktierung erfolgt erfindungsgemäß in der Weise, dass Licht in das lichtleitende Element eingekoppelt werden kann und das von dem Testfeld weggeleitete Licht von der Analyseeinheit detektiert wird. Durch die Ankopplung des lichtleitenden Elementes an eine Stechhilfe mit Analyseeinheit ist eine Positionierung des Testfeldes relativ zur Messoptik automatisch gegeben, so dass das Testfeld stets in einer Messposition vorliegt und vermessen werden kann. Eine optische Kontaktierung zwischen einem Analysegerät und einem Testelement wird beispielsweise in dem Dokument WO 01/48461 beschrieben, auf das an dieser Stelle Bezug genommen wird. Eine so ausgestaltete Stechhilfe ist als vollwertiges Analysesystem anzusehen, so dass der Benutzer nur ein einziges Gerät zur Durchführung der Analyse handhaben muss.

Zur Messung eines Analyten setzt der Benutzer das Analysesystem zunächst auf einen Körperteil, z. B. der Fingerkuppe auf, wo er den Stechvorgang auslöst. Durch die Antriebseinheit wird nun die Lanzette in Einstichrichtung bewegt, so dass die Lanzettenspitze in die Fingerkuppe eindringt und dort eine Wunde erzeugt. Das aus der Wunde austretende Blut wird anschließend mit dem Testfeld, das im wesentlichen im Bereich der Einstichstelle positioniert ist, in Kontakt gebracht.

Erfindungsgemäss ist das Testfeld fest mit dem Lichtleiter verbunden und es kann z. B. durch die oder eine weitere Antriebseinheit in der Stechhilfe zusätzlich zur Lanzette ebenfalls der Lichtleiter bewegt werden, so dass das Testfeld nach dem Stechvorgang direkt an die Wunde herangeführt werden kann. Auf diese Weise kann eine verbesserte Probenaufnahme erzielt werden. Verzichtet man hingegen auf diese zusätzlichen Maßnahmen, zeigen Experimente, dass auch ohne zusätzliche Führung des Testfeldes eine hinreichend gute Probenaufnahme durch das System gegeben ist. Dies zeigt sich insbesondere bei einer vorteilhaften Ausführungsform, bei der die Lanzette in einer Ebene senkrecht zur Einstichrichtung unmittelbar neben den Lichtleiter und oder dem Testfeld angeordnet ist. Einstichstelle sowie Mess- und oder Probenaufnahmeort liegen somit in der Ebene senkrecht zur Einstichrichtung in unmittelbarer Nähe zueinander. Nach dem Stechvorgang ist folglich das Testfeld sowie das Ende des Lichtleiters im wesentlichen direkt an der Wunde positioniert. Die Vermessung des Testfeldes erfolgt anschließend direkt am Probenaufnahmeort. Hierbei bleiben dem Benutzer vorteilhafterweise jegliche zusätzlichen Handhabungsschritte erspart, sofern eine Auswertung unmittelbar durch die Stechhilfe geleistet wird. Dem Benutzer wird das Analyseergebnis dann direkt über einen Display der Stechhilfe mitgeteilt. Nach Gebrauch des Analysesystems wird das System aus der Stechhilfe entfernt bzw. ausgetauscht.

Natürlich sind auch Ausführungsformen denkbar, bei denen ein separater Anschluss des Systems an eine Analyseeinheit erfolgt. Ist die Analyseeinheit nicht in der Stechhilfe integriert, kann z. B. die Stechhilfe mit dem System an ein hierfür vorgesehenes Analysegerät angeschlossen werden. Hierbei verfügt die Stechhilfe über einen entsprechenden Kopplungsmechanismus, der die Analyseeinheit via Stechhilfe mit dem System optisch kontaktiert. Es ist natürlich auch denkbar, dass das System, nachdem es aus der Stechhilfe entnommen wurde, direkt an eine hierfür geeignete Analyseeinheit angekoppelt werden kann. Bestrebungen sind es jedoch, dem Benutzer separate Handhabungsschritte weitestgehend zu ersparen, so dass bei einer bevorzugten Ausführungsform der Benutzer das Analyseergebnis nach dem Auslösen des Stechvorgangs direkt am hoch integriertem Analysesystem ablesen kann.

Hierfür enthält die Stechhilfe in einer bevorzugten Ausführungsform eine Mess- und Auswerteelektronik wie sie im Stand der Technik hinlänglich bekannt ist. An die Mess- und Auswerteelektronik angeschlossen, ist z. B. eine Leuchtdiode (LID), die als Lichtsender das Primärlicht in das lichtleitende Element des Systems einkoppelt. Das vom System weggeleitete Sekundärlicht wird z. B. mittels einer Photodiode, die als Detektor Bestandteil der optischen Messeinrichtung ist, erfasst.

Um das Handling für den Benutzer weiter zu vereinfachen, kann eine Stechhilfe eine Vielzahl von Systemen beinhaltet, die in einem Vorratsbehälter magaziniert vorliegen und sukzessive dem Benutzer zum Gebrauch zur Verfügung stehen. Hierbei ist die Integration vielfältiger Ausführungsformen des erfindungsgemäßen Systems, wie sie bereits beschrieben wurden, denkbar, bei denen das System z. B. über eine Mehrzahl von Lanzetten verfügt. Eine Magazinierung erfolgt dann entsprechend, so dass z. B. auch ausschließlich eine Magazinierung von einzelnen Elementen des Systems, wie z. B. Lanzetten, möglich ist. Vorzugsweise wird das System oder einzelne Elemente hieraus nach dem Gebrauch innerhalb des Vorratsbehälters oder einem weiteren Abfallbehältnis wieder remagaziniert, so dass eine komfortable Beseitigung gebrauchter Systeme/Elemente nach jedem Stechvorgang gewährleistet wird. Auf diese Weise wird eine sichere und hygienische Entsorgung sowie Handling des Systems gewährleistet. Eine Magazinierung in einer Stechhilfe wie beschrieben kann auf vielfältige Weise erfolgen, und ist im Stand der Technik hinlänglich bekannt.

Das System kann im Rahmen der Erfindung auf unterschiedliche Weise verwirklicht werden. In einer bevorzugten Ausführungsform ist die Lanzettenspitze und das Testfeld konzentrisch zueinander angeordnet, wobei z. B. die Lanzette zumindest zum Teil vom lichtleitenden Element umgeben wird und bevorzugt innerhalb einer hohlen Lichtleiterfaser geführt wird. Durch die Integration der Lanzette in die Hohlfaser kann die Lanzette vorteilhafterweise relativ zum Lichtleiter in der Weise in- und entgegen der Einstichrichtung bewegt werden, dass die Lanzettenspitze ausschließlich bei einem Stechvorgang aus der Hohlfaser hervortritt. Die Lanzettenspitze wird folglich nach bzw. vor dem Einstich schützend von der Hohlfaser umgeben. Zusätzliche Funktionen, wie z. B. Sterilschutz der Lanzettenspitze, sowie einen Schutz des Benutzers vor Verletzungen an der Lanzettenspitze können in das System auf diese Weise mühelos integriert werden. Um die Sterilität der Lanzettenspitze im System zu gewährleisten, können natürlich auch zusätzliche Maßnahmen - wie es im Stand der Technik bereits bekannt sind - ergriffen werden. Beispielsweise wird an dieser Stelle auf das Dokument WO 01/66010 Bezug genommen, das ein Sterilschutz für Lanzetten offenbart, bei dem die Lanzettenspitzen in einem Elastomer eingebettet werden.

Darüber hinaus sind auch komplementäre Ausführungsformen denkbar, bei denen die Lanzette zumindest zum Teil das lichtleitende Element umgibt, und der Lichtleiter z. B. innerhalb einer hohlen Lanzette angeordnet ist. Vorteilhafter Weise verfügt die Stechhilfe dann - wie oben bereits ausgeführt - über eine Antriebseinheit für das lichtleitende Element.

Nachfolgend werden anhand der Figuren spezifische Ausführungsformen erläutert:
- Figur 1:: System mit einer lichtleitenden Hohlfaser
- Figur 2:: System mit einer Hohlkanüle
- Figur 3:: Analy sesystem
- Figur 4:: System mit Testfeldband

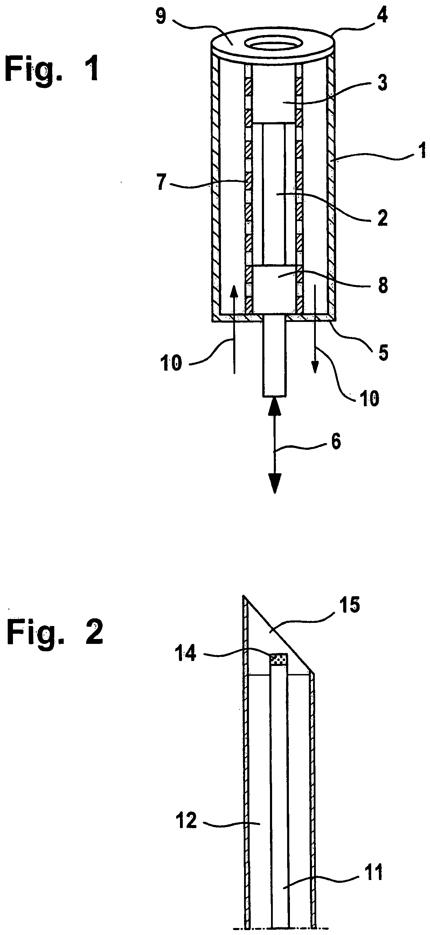

Figur 1 zeigt ein System, bei dem eine lichtleitende Hohlfaser (1) konzentrisch um eine Lanzette (2) angeordnet ist. Die Hohlfaser verfügt über ein proximales Ende (5), in das Licht einer Analyseeinheit (nicht gezeigt) ein- bzw. ausgekoppelt werden kann. An einem distalem Ende (4) des Lichtleiters weist das Element eine Reagenzschicht (9) auf, die auf den Lichtleiter durch Aufkleben oder Aufpolymerisieren aufgebracht wurde und mit diesem fest verbunden ist. Der Stechort und das Testfeld sind somit konzentrisch zueinander angeordnet, wobei das Testfeld die Lanzettenspitze ringförmig umgibt. Der Außendurchmesser des Systems beträgt im gezeigten Beispiel einige wenige Millimeter. Die Lanzettenspitze (nicht gezeigt) ist in einen Sterilschutz (3) eingebettet, der die Sterilität der Lanzette gewährleistet. Beim Ausführen des Stechvorgangs durchdringt die Lanzettenspitze den elastisch ausgebildeten Sterilschutz, so dass die Lanzettenspitze aus dem Sterilschutz austritt und während des Stechvorgangs über das distale Ende (4) des Lichtleiters (1) und über das Testfeld (9) hinausragt. Die Lanzettenspitze wird nach dem Stechvorgang wieder in ihrer Ruheposition in die Hohlfaser zurückgezogen, wo sie von dem Sterilschutz (3) schützend umgeben wird. Das System weist weiterhin ein aus Plastik geformtes Führungselement (8) auf, das die Lanzette (2) innerhalb der Hohlfaser während des Stechvorgangs sicher und vibrationsarm führt. Die Lanzette wird hierfür in oder entgegen der Einstichrichtung (6) relativ zum Lichtleiter bewegt. Wurde Probe auf das Testfeld (9) aufgetragen, wird das Licht (10) zunächst zum Testfeld hin geleitet, wo es mit dem in der Probe enthaltenen Analyt-Reagenzkomplex wechselwirkt. Anschließend wird das Licht diffus vom Testfeld in die Hohlfaser reflektiert oder als Fluoreszenz emittiert und über Totalreflektion innerhalb des Lichtleiters zu einem Detektor (nicht gezeigt) geleitet. Eine optisch detektierbare Veränderung des Testfeldes (4) durch einen Analyten kann somit erfasst und vermessen werden. Beispielsweise dient ein derartiges System zur Analyse eines Glucosekonzentration in einer Probe.

Das System ist als Einmalartikel vorgesehen. Unter dieser Bedingung beinhaltet das Testfeld (4) in der Regel ein Reagenz, das irreversible mit einem Analyten der Probe reagiert. Nach einem einmaligen Gebrauch wird das System verworfen.
Figur 2 zeigt eine komplementäre Ausführungsform des oben beschriebenen Systems. Das System weist eine Hohlkanüle (12) auf, innerhalb der sich ein lichtleitendes Element (11) befindet. Das distale Ende des lichtleitenden Elementes ist ebenfalls mit einem analytspezifischen Reagenz (14) beschichtet und bildet somit das Testfeld aus. Die Hohlkanüle verfügt über eine Spitze, die zur Erzeugung einer Hautöffnung geeignet ist. In ihrem oberen Bereich weist die Hohlkanüle eine Öffnung (15) auf. Wird durch die Hohlkanüle eine Wunde in einen Körperteil eines Patienten erzeugt, ermöglicht die Öffnung (15) einen Kontakt des distalen Endes des Lichtleiters und somit des Testfeldes mit der Probe. Es zeigt sich, dass durch eine zusätzliche Führung des lichtleitenden Elementes relativ zur Hohlkanüle eine Probenaufnahme durch das Testfeld erleichtert werden kann. Hierfür wird nach dem Stechvorgang das distale Ende des Lichtleiters aus der Öffnung (15) geschoben bis es über die Spitze der Lanzette hinausragt. Ein Kontakt des Testfeldes mit dem Blut des Patienten ist somit problemlos möglich.

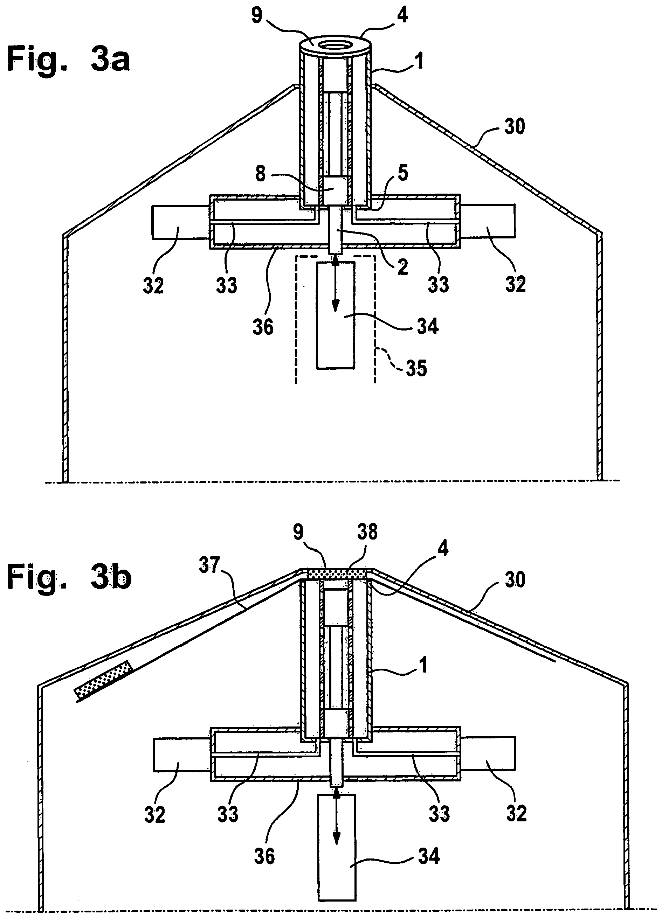

Figur 3 zeigt schematisiert ein Analysesystem, das in einer Stechhilfe realisiert ist. Die Stechhilfe verfügt zur Auswertung des Systems über eine Analyseeinheit (32) sowie über eine Antriebseinheit (34) zur Ankopplung der Lanzette an die Stechhilfe.

Figur 3 zeigt im wesentlichen eine Integration eines zu Figur 1 analogen Systems, das über eine lichtleitende Hohlfaser (1) die konzentrisch um die Lanzette (2) angeordnet ist, verfügt. Zum Ausführen eines Stechvorgangs, wird die Lanzette (2) durch den Lanzettenantrieb (34) in Einstichrichtung bewegt, so dass die Lanzettenspitze (nicht gezeigt), über das distale Ende der Hohlfaser hinausragt und eine Wunde, in einer dort platzierten Fingerkuppe, erzeugen kann. Anschließend wird die Lanzette durch die Antriebseinheit wieder zurückgezogen, wobei das Führungselement (8) für eine vibrationsarme Ausführung des Stechvorgangs sorgt. Zur Analyse des Testfeldes wird am proximalen Ende (5) der Hohlfaser Licht durch eine Analyseeinheit (32) eingekoppelt. Hierfür verfügt die Analyseeinheit (32) über einen Lichtsender, der über weitere zusätzliche lichtleitende Elemente (33) mit dem System gekoppelt ist. Hierbei werden die lichtleitenden Elemente (33), die permanent in der Stechhilfe angeordnet sind, an die lichtleitende Hohlfaser des als Einmalartikel vorgesehenen Systems optisch angekoppelt. Das Licht kann so durch die lichtleitende Hohlfaser (1) zu dem distalen Ende (4) der Hohlfaser geleitet werden und somit zu dem Testfeld (9), gemäß dem in Figur 1 gezeigten System. Durch die Bestrahlung des Testfeldes, wird in Abhängigkeit des in einer Probe enthaltenen Analyten, eine Fluoreszenzstrahlung angeregt. Das auf diese Weise emittierte Licht wird über das lichtleitende Element (33) aus der Hohlfaser ausgekoppelt und zu einem Detektor der Analyseeinheit (32) geleitet.

Des weiteren ist vorstellbar, dass eine zusätzlich Antriebseinheit (35) vorgesehen ist, die an die Hohlfaser ankoppelt und diese ebenfalls zur verbesserten Probenaufnahme nach dem Stechvorgang in Einstichrichtung an die Blutaustrittsstelle führt. Ist das System dann z. B. im wesentlichen vollständig innerhalb des Gehäuses (30) der Stechhilfe positioniert tritt während des Stechvorgangs zunächst ausschließlich die Lanzette aus dem Gehäuse (30) aus. Nach dem Einstich wird dann die Hohlfaser mit ihrem distalen Ende entsprechend über den Antrieb (35) zur Wunde hingeführt, so dass eine Probenaufnahme erleichtert wird.

Im gezeigten Beispiel wird jedoch auf die schematisch angedeutete Antriebseinheit (35) für die Hohlfaser verzichtet, so dass stattdessen die Hohlfaser ortsfest in einen Halter (36) innerhalb des Analysesystems positioniert ist. Nach einmaligen Gebrauch des erfindungsgemäßen Systems, kann dieses von dem Benutzer aus dem Halter (36) in der Stechhilfe entnommen und ausgetauscht werden. Beim erneuten Einführen des Systems in den Halter (36) erfolgt automatisch eine optische Ankopplung der Hohlfaser an das oder die lichtleitenden Elemente (33), sowie eine mechanische Ankopplung an die Antriebseinheit- oder Einheiten.

Die Ankopplung der Antriebseinheiten (34) und/oder (35) an die Lanzette bzw. an das lichtleitende Element, kann dabei prinzipiell auf vielfältige Weise erfolgen und ist in einer vorteilhaften Ausführungsform so ausgestaltet, dass eine Bewegung der Elemente in als auch gegen die Einstichrichtung erfolgen kann. Beispielsweise kann eine derartige Ankopplung der Antriebseinheit-/en durch eine formschlüssige Kopplung an die Lanzette und/oder an die Hohlfaser realisiert werden, die entsprechend ausgebildet sind. Eine derartige formschlüssige Kopplung, die insbesondere für integrierte Systeme geeignet ist, wird beispielsweise in dem Dokument DE 103 02 501.4 beschrieben, auf das an dieser Stelle inhaltlich Bezug genommen wird.

Figur 3b zeigt ein nicht erfindungsgemäßes Analysesystem bei dem das lichtleitende Element in Form einer Hohlfaser (1) reversibel an dem Testfeld (38) positioniert ist. Die Ankopplung des nicht erfindungsgemäßen Systems an das Analysegerät erfolgt analog zu den in Figur 3a gezeigten Ausführungsformen. Das Analysesystem verfügt somit ebenfalls in seinem Gehäuse (30) über eine Analyseeinheit (32) sowie eine für die Lanzette vorgesehene Antriebseinheit (34). Die Hohlfaser (1) wird über den Halter (36) ortsfest im Analysesystem gehalten. Das distale Ende (4) der Hohlfaser ist an einem transparenten Trägerband (37) reversibel positioniert, so dass das Trägerband entlang des distalem Endes des Lichtleiters beweglich geführt werden kann. Das Trägerband weist in regelmäßigen Abständen Bereiche auf, die mit einem Reagenz beschichtet sind und somit das Testfeld (9) ausbilden. Hierbei liegt zunächst das dünne Trägerband zwischen dem distalen Ende des Lichtleiters und der Haut. Der Stich erfolgt entweder direkt durch das Trägerband oder durch ein vorgeformtes Loch darin. Direkt nach dem Stich wird das Trägerband weiterbewegt, so dass ein Testfeld zwischen Lichtleiter und Haut zu liegen kommt und dort die austretende Probe aufnehmen kann. Eine verbesserte Probenaufnahme des Systems kann folglich auch durch eine geführte Bewegung eines Testfeldes erfolgen, ohne dass dieses fest mit dem Lichtleiter verbunden ist. Alternativ kann auch bereits während des Stechvorganges ein auf dem Trägerband positioniertes Testfeld zwischen dem Lichtleiter und Haut liegen. Der Stich erfolgt dann zweckmäßigerweise durch ein Loch im Testfeld, oder direkt durch das Testfeld hindurch.

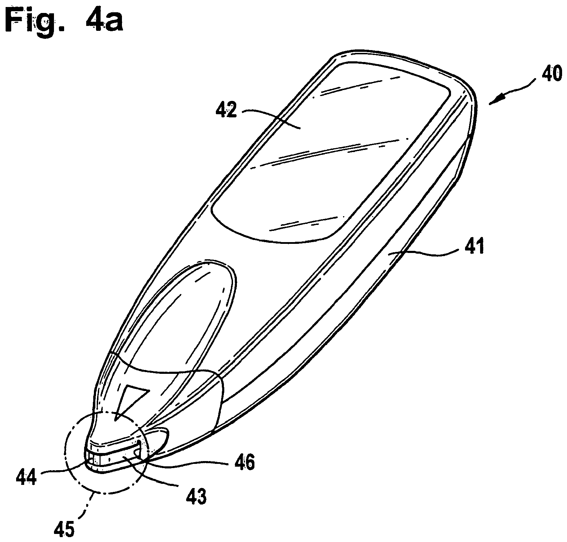

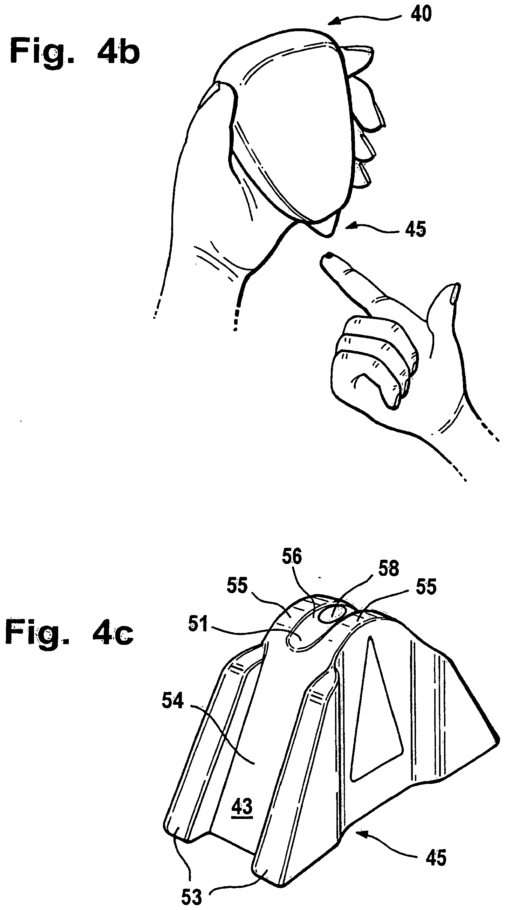

Die Hohlfaser in Figur 3b muss vorteilhafterweise nicht ausgetauscht werden und kann als festes Bestandteil des Analysesystems verwendet werden. Ebenso ist es denkbar die Lanzette zu mehrmaligem Gebrauch vorzusehen, wobei jedoch mit einer Abnutzung der Lanzettenspitze üblicherweise gerechnet werden muss. Im Analysesystem würden dann zum einmaligen Gebrauch zunächst nur die Testfelder vorgesehen sein. Aufgrund der Verwendung eines Teststreifenbandes - wie es anhand von Figur 4 noch näher erläutert wird - kann das Band und somit das Testfeld nach Gebrauch entlang dem distalen Ende der Hohlfaser einfach weiter transportiert werden bis ein unbenutztes Testfeld erneut zur Blutaufnahme zur Verfügung steht. Der Benutzer kann auf diese Weise eine Blutanalyse vornehmen, ohne vor jedem Gebrauch Elemente des Analysesystems auswechseln zu müssen. Sind das Testfeldband sowie die Lanzette zur mehrfachen Verwendung geeignet, wird dem Benutzer eine komfortable und unauffällige Handhabung des Analysesystems ermöglicht.

Figur 4 a bis 4c zeigen ein Analysesystem, bei dem mehrere Testfelder auf einer Bandkassette angeordnet sind. Das Analysesystem (40) verfügt über ein Gehäuse (41), das in einer für den Benutzer leicht zu handhabenden Form ausgebildet ist. In einem spitz ausgeprägten vorderem Ende (45) des Analysesystems weist das Gehäuse eine Öffnung (46) auf, aus der ein Testfeldband (43) austritt. Zur Realisierung eines Analysesystems mit einem Testfeldband wird auf das Dokument EP 02026242.4 verwiesen, auf das an dieser Stelle inhaltlich Bezug genommen wird. Das Testfeldband (43) besitzt in periodischen Abständen Bereiche (44), die mit einer Reagenzchemie beschichtet sind und somit ein Testfeld (44) ausbilden. Zur Ausführung eines Stechvorgangs wird das Analysesystem (40) mit dem vorderen Ende (45) auf die Fingerkuppe eines Patienten aufgesetzt. Das Testfeldband ist hierbei so positioniert, dass ein Testfeld (44) direkt am vorderen Ende (45) des Analysesystems aufliegt.

Zur näheren Veranschaulichung wird das vordere Ende (45) des Analysesystems vergrößert in Figur 4(c) dargestellt. Hierbei ist zu erkennen, dass das Testfeldband innerhalb von Führungsschienen (53) des vorderen Endes (45) geführt wird. Das Testfeldband weist in dem Bereich, in dem das Testfeld ausgebildet ist, eine Ausnehmung (56) auf. Die Testchemie des Testfeldes ist in einem äußeren Bereich (55), der die Ausnehmung (56) umgibt, aufgebracht. In den verbleibenden Bereichen (54) des Testfeldbandes, in dem kein Testfeld vorgesehen ist, ist das Band einstückig ausgebildet. Hierdurch kann die Führung des Bandes innerhalb des Analysesystems stabilisiert werden. Der vordere Bereich (45) des Analysesystems weist weiterhin an seinem vorderen Ende ein Loch (58) auf, das in einer Auflagefläche (51) eingebracht ist. Zum Ausführen des Stechvorgangs tritt eine Lanzette durch das Loch (58) und die Ausnehmung (56) des Testfeldes aus, so dass eine Wunde in einer dort aufliegenden Fingerkuppe erzeugt werden kann. Nach dem Stechvorgang wird das aus der Wunde austretende Blut von den Testfeldbereichen (55) aufgenommen, wo es mit dem im Testfeld enthaltenem Reagenz entsprechend wechselwirkt. Die Auflagefläche (51), auf der das Testfeld während des Stech- und Messvorgangs aufliegt, sowie das Testfeldband sind optisch transparent ausgebildet. Mit Hilfe von zwei lichtleitenden Elementen, die direkt an der Auflagefläche (51) innerhalb des Analysensystem angeordnet sind, kann eine Vermessung des Testfeldes am Probenaufnahmeort erfolgen. Nach der Analyse der Probe wird das Testfeldband entsprechend einem Bandkassettenprinzip, wie es in dem Dokument EP 02026242.4 beschrieben ist weitertransportiert bis das nächste Testfeld oberhalb der Auflagenfläche (51) positioniert ist. Das Analysesystem ist nun bereit erneut einen Stech- und Messvorgang durchzuführen. Hierfür verfügt das System beispielsweise über eine Vielzahl von Lanzetten. Das Ergebnis der Analyse wird dem Benutzer über den Display (12) mitgeteilt.

## Patentansprüche

1. Blutentnahmesystem beinhaltend
eine Stechhilfe,
ein System zur Analyse einer zu untersuchenden Probe beinhaltend
- ein Testfeld mit einem Reagenz (14),
- mindestens ein lichtleitendes Element (11) mit einem ersten distalen Ende und
- einem zweiten proximalen Ende, in das Licht eingekoppelt werden kann, so dass Licht von dem zweiten Ende zu dem Testfeld hingeleitet und vom Testfeld durch das selbe oder einem weiterem lichtleitenden Element wieder weitergeleitet wird, sowie
- eine Lanzette (12) mit einer Lanzettenspitze, die in der Weise im Bereich des distalen Endes sowie des Testfeldes angeordnet ist, dass
- die Lanzettenspitze während eines Stechvorgangs über das distale Ende des Lichtleiters sowie über das Testfeld hinausragt, und dass das Reagenz des Testfeldes bei Kontakt mit einem in der Probe enthaltenen Analyten im Wesentlichen irreversibel reagiert, so dass eine optisch detektierbare Veränderung im Testfeld bewirkt wird und das mindestens eine lichtleitende Element mit seinem distalen Ende fest mit dem Testfeld verbunden ist, sodass das System zur Analyse einer zu untersuchenden Probe nur zur einmaligen Verwendung geeignet ist, wobei das System zur Analyse einer zu untersuchenden Probe in der Stechhilfe verwendet wird und die Stechhilfe eine Antriebseinheit für die Lanzette beinhaltet.

2. System gemäß Anspruch 1, das eine Vielzahl von Lanzetten aufweist.

3. System gemäß Anspruch 1,
- bei dem das lichtleitende Element zumindest zum Teil von der Lanzette umgeben wird.

4. System gemäß Anspruch 3,
- bei dem das lichtleitende Element innerhalb einer hohlen Lanzette angeordnet ist.

5. System gemäß Anspruch 1,
- bei dem die Lanzette und das lichtleitende Element konzentrisch zueinander angeordnet sind.

6. System gemäß Anspruch 1,
- bei dem die Lanzette und das lichtleitende Element in einer Ebene senkrecht zur Einstichrichtung in unmittelbarer Nähe zueinander angeordnet sind.

7. System gemäß Anspruch 1,
- bei dem die Lanzettenspitze in einen Sterilschutz eingebettet ist.

8. System gemäß Anspruch 1,
- das zur Bestimmung einer Glucosekonzentration aus Blut geeignet ist.

9. System gemäß Anspruch 1,
- das mit einer Analyseeinheit eines Analysegerätes optisch kontaktiert werden kann, so dass Licht in das lichtleitende Element ein- bzw. ausgekoppelt wird.

10. System gemäß Anspruch 1,
- bei dem die Stechhilfe eine Analyseeinheit beinhaltet, die mit dem lichtleitenden Element in der Weise optisch kontaktiert ist, dass Licht in das lichtleitende Element eingekoppelt und das von dem Testfeld weggeleitete Licht von der Analyseeinheit detektiert werden kann.

11. System gemäß Anspruch 1,
- bei dem die Stechhilfe an eine Analyseeinheit angekoppelt werden kann, so dass Licht in das lichtleitende Element eingekoppelt und das von dem Testfeld weitergeleitete Licht von der Analyseeinheit detektiert werden kann.

12. System gemäß Anspruch 1,
- bei dem die Stechhilfe eine Antriebseinheit für das lichtleitende Element beinhaltet.

13. System gemäß Anspruch 1 oder 10,
- bei dem die Stechhilfe eine Antriebseinheit zum Transport des Testelements beinhaltet.

14. System gemäß Anspruch 1,
- das in einem Magazin der Stechhilfe positioniert wird, in dem sich eine Vielzahl von Systemen befinden.

## Claims

1. Blood collection system containing
a puncturing aid,
a system for analysing a sample to be examined, containing
- a test field with a reagent (14),
- at least one light-guiding element (11) comprising a first distal end and
- a second proximal end, into which light can be coupled such that light is guided from the second end to the test field and, from the test field, guided again through the same light-guiding element or a further light-guiding element, and
- a lancet (12) with a lancet tip, which is arranged in the region of the distal end and of the test field in such a way that
- the lancet tip protrudes beyond the distal end of the light guide and the test field during a puncturing process and that the reagent of the test field reacts substantially irreversibly upon contact with an analyte contained in the sample such that an optically detectable change in the test field is effected and the at least one light-guiding element is securely connected, with the distal end thereof, to the test field,
such that the system for analysing a sample to be examined is only suitable for single use, wherein the system for analysing a sample to be examined is used in the puncturing aid and the puncturing aid contains a drive unit for the lancet.

2. System according to Claim 1, which has a multiplicity of lancets.

3. System according to Claim 1,
- wherein the light-guiding element is at least partly surrounded by the lancet.

4. System according to Claim 3,
- wherein the light-guiding element is arranged within a hollow lancet.

5. System according to Claim 1,
- wherein the lancet and the light-guiding element are arranged concentrically in relation to one another.

6. System according to Claim 1,
- wherein the lancet and the light-guiding element are arranged in the direct vicinity of one another in a plane perpendicular to the piercing direction.

7. System according to Claim 1,
- wherein the lancet tip is embedded in a sterile protection.

8. System according to Claim 1,
- which is suitable for determining a glucose concentration from blood.

9. System according to Claim 1,
- which can be optically contacted with an analysis unit of an analysis device such that light is coupled into, or decoupled from, the light-guiding element.

10. System according to Claim 1,
- wherein the puncturing aid contains an analysis unit which is optically contacted to the light-guiding element in such a way that light can be coupled into the light-guiding element and the light guided away from the test field can be detected by the analysis unit.

11. System according to Claim 1,
- wherein the puncturing aid can be coupled to an analysis unit such that light can be coupled into the light-guiding element and the light guided away from the test field can be detected by the analysis unit.

12. System according to Claim 1,
- wherein the puncturing aid contains a drive unit for the light-guiding element.

13. System according to Claim 1 or 10,
- wherein the puncturing aid contains a drive unit for transporting the test element.

14. System according to Claim 1,
- which is positioned in a cartridge of the puncturing aid, in which a multiplicity of systems are situated.

## Revendications

1. Système de prélèvement de sang, comprenant
un dispositif autopiqueur
un système d'analyse d'un échantillon à examiner, comprenant
- un champ d'essai comportant un réactif (14),
- au moins un élément conducteur de lumière (11) pourvu d'une première extrémité distale et
- d'une deuxième extrémité proximale, dans laquelle on peut injecter de la lumière, faisant en sorte que la lumière soit acheminée de la deuxième extrémité vers ledit champ d'essai pour ensuite être conduite à travers ce dernier ou bien de manière à quitter le champ d'essai par un autre élément conducteur de lumière, ainsi qu'une
- lancette (12) comportant une pointe de lancette laquelle est disposée au niveau de ladite extrémité distale et dudit champ d'essai de manière à ce que
- la pointe de lancette dépasse, lors du processus de piqûre, l'extrémité distale du conducteur de lumière ainsi que ledit champ d'essai, et que ledit réactif du champ d'essai subisse, lorsqu'il entre en contact avec un analyte contenu dans l'échantillon, une réaction sensiblement irréversible, faisant en sorte qu'un changement optiquement détectable se produise au sein du champ d'essai, l'extrémité distale de l'au moins un élément conducteur de lumière étant solidaire audit champ d'essai,
le système d'analyse d'un échantillon à examiner étant ainsi réservé à un usage unique, le système d'analyse d'un échantillon à examiner étant mis en oeuvre au sein dudit autopiqueur, et le ledit autopiqueur comprenant une unité d'entraînement pour ladite lancette.

2. Système selon la revendication 1, comportant une pluralité de lancettes.

3. Système selon la revendication 1,
- ledit élément conducteur de lumière étant au moins partiellement entouré de ladite lancette.

4. Système selon la revendication 3,
- ledit élément conducteur de lumière étant disposé à l'intérieur d'une lancette creuse.

5. Système selon la revendication 1,
- ladite lancette et ledit élément conducteur de lumière étant en disposition concentrique l'un par rapport à l'autre.

6. Système selon la revendication 1,
- ladite lancette et ledit élément conducteur de lumière étant disposés dans un plan perpendiculaire au sens de la piqûre, à proximité immédiate l'un de l'autre.

7. Système selon la revendication 1,
- la pointe de lancette étant incorporée dans un dispositif de protection stérile.

8. Système selon la revendication 1,
- permettant de déterminer une concentration de glucose dans le sang.

9. Système selon la revendication 1,
- pouvant être mis en contact optique avec une unité d'analyse d'un appareil d'analyse, pour faire en sorte que la lumière soit injectée dans ledit élément conducteur de lumière et qu'elle en soit extraite.

10. Système selon la revendication 1,
- ledit autopiqueur comprenant une unité d'analyse laquelle est mis en contact optique avec ledit élément conducteur de lumière de manière à ce que de la lumière soit injectée dans ledit élément conducteur de lumière et que la lumière puisse être détectée par ladite unité d'analyse après avoir quitté le champ d'essai par un conducteur.

11. Système selon la revendication 1,
- ledit autopiqueur pouvant être couplé à une unité d'analyse, permettant ainsi d'injecter de la lumière dans ledit élément conducteur de lumière et de détecter la lumière, après avoir quitté le champ d'essai, au moyen de ladite unité d'analyse.

12. Système selon la revendication 1,
- ledit autopiqueur comprenant une unité d'entraînement destinée audit élément conducteur de lumière.

13. Système selon les revendications 1 ou 10,
- ledit autopiqueur comprenant une unité d'entraînement destinée à transporter l'élément d'essai.

14. Système selon la revendication 1,
- lequel est positionné dans un chargeur appartenant audit autopiqueur et renfermant une pluralité de systèmes.
